# DEMANDE DE BREVET EUROPEEN

(11) **EP 2 520 296 A1**
(43) Date de publication de la demande: **07.11.2012**
(21) Numéro de dépôt: 12166348.8
(22) Date de dépôt: 02.05.2012
(51) Int. Cl.: A61K 31/403, A61K 45/06, A61K 31/445, A61K 31/27, A61K 31/55, A61K 9/20, A61P 25/16, A61P 25/28

(54) **Nouvelle association entre le 4-{3-[cis-hexahydrocyclopenta[c]pyrrol-2(1h)-yl]propoxy}benzamide et un inhibiteur de l'acetylcholinesterase et les compositions pharmaceutiques qui la contiennent**

(30) Priorité: 02.05.2011 FR 1101347
(71) Demandeur: Les Laboratoires Servier, 92284 Suresnes Cedex (FR)
(72) Inventeur: Trocme-Thibierge, Caryn, 92270 BOIS COLOMBES (FR); Sors, Aurore, 75015 PARIS (FR); Keime Guibert, Florence, 275851 SINGAPOUR (SG)

(57) **Abrégé**

Association entre le 4-{3-[*ci*s-hexahydrocyclopenta[*c*]pyrrol-2(1*H*)-yl]propoxy}benzamide de formule (I) : 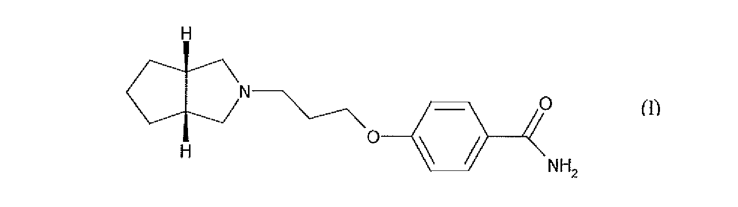 ou un de ses sels d'addition à un acide ou à une base pharmaceutiquement acceptable et un inhibiteur de l'acétylcholinesterase.

Médicaments.

## Description

La présente invention concerne une nouvelle association entre le *4-*{3-[*cis-*hexahydrocyclopenta[*c*]pyrrol-2(1*H*)-yl]propoxy}benzamide de formule (I): 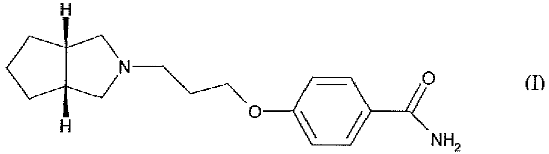 ou un de ses sels d'addition à un acide ou à une base pharmaceutiquement acceptable, et un inhibiteur de l'acétylcholinesterase pour l'obtention de compositions pharmaceutiques utiles dans le traitement des troubles cognitifs associés au vieillissement cérébral et aux maladies neurodégénératives.

Le 4-{3-[*cis*-hexahydrocyclopenta[*c*]pyrrol-2(1*H*)-yl]propoxy}benzamide présente la particularité d'interagir avec les systèmes histaminergiques centraux in vivo. Ces propriétés lui confèrent une activité dans le système nerveux central et plus particulièrement dans le traitement des déficits cognitifs associés au vieillissement cérébral et aux maladies neurodégénératives.

Le 4-{3-[*cis*-hexahydrocyclopenta[*c*]pyrrol-2(1*H*)-yl]propoxy}benzamide, sa préparation et son utilisation en thérapeutique ont été décrits dans la demande de brevet WO2005/089747.

La demanderesse a présentement découvert que le 4-{3-[*cis*-hexahydrocyclopenta[*c*]pyrrol -2(1*H*)-yl]propoxy}benzamide de formule (I), ou ses sels d'addition à un acide ou à une base pharmaceutiquement acceptable, utilisé en association avec un inhibiteur de l'acétylcholinesterase, possédait des propriétés intéressantes pour le traitement des troubles cognitifs associés au vieillissement cérébral et aux maladies neurodégénératives.

Les maladies neurodégénératives liées au vieillissement cérébral telles que la maladie d'Alzheimer sont caractérisées par des troubles de la mémoire et des dysfonctionnements cognitifs. Les troubles cognitifs sont généralement associés à une diminution de la capacité des neurones à synthétiser et à libérer certains neurotransmetteurs. De plus, on observe une perte progressive de la plasticité synaptique et des processus neuronaux, cette perte neuronale étant accélérée dans certaines régions spécifiques du cerveau. Parmi les divers neurotransmetteurs, l'histamine et l'acétylcholine centrales jouent un rôle crucial dans le contrôle des fonctions cognitives (Witkin and Nelson, Pharmacol. & Therap., 2004, 103, 1-20) et il a été montré que leurs taux diminuaient fortement dans le cerveau des patients atteints par la maladie d'Alzheimer par rapport à ceux observés chez des personnes âgées saines (Panula et al, Neuroscience, 1998, 82(4), 993-997).

Les récepteurs histaminergiques de type H₃, particulièrement abondants dans le système nerveux central, sont des modulateurs principalement présynaptiques de la transmission nerveuse et sont présents dans divers circuits neuronaux en rapport avec la cognition (Blandina et al, Learn Mem., 2004, 11(1), 1-8). Ils agissent en régulant de manière négative la libération de neurotransmetteurs tels que l'histamine, l'acétylcholine, la sérotonine, la noradrénaline et la dopamine. Etant donné que les neurones histaminergiques semblent être largement épargnés dans la maladie d'Alzheimer, les composés antagonistes ou agonistes inverses des récepteurs H₃ pourraient ouvrir la voie à de nouveaux traitements des troubles cognitifs liés au vieillissement cérébral.

A l'inverse, on observe une dégénérescence progressive des neurones cholinergiques au cours de la maladie d'Alzheimer. Les inhibiteurs de l'acétylcholinestérase tels que le donépézil sont couramment utilisés dans le traitement symptomatique de la maladie d'Alzheimer afin de limiter la diminution des taux d'acétylcholine dans le cerveau en bloquant l'action de l'acétylcholinestérase. Il a été montré que les inhibiteurs d'acétylcholinestérase, tout comme les antagonistes /agonistes inverses des récepteurs H₃, permettaient d'améliorer les propriétés cognitives dans différents modèles animaux de mémoire épisodique et de mémoire de travail (Esbenshade et al, Br. J. Pharmacol., 2008, 154(6), 1166-1181; Yuede et al, Behav. Pharmacol., 2007, 18(5-6), 347-363). L'amélioration des fonctions cognitives peut donc reposer sur deux types de stratégie, ciblant soit l'histamine, soit l'acétylcholine.

De façon surprenante, la présente invention a montré que les effets des inhibiteurs de l'acétylcholinesterase sont potentialisés par ceux du 4-{3-[*cis-*hexahydrocyclopenta[*c*]pyrrol-2(1*H*)-yl]propoxy}benzamide ou de ses sels d'addition à un acide ou à une base pharmaceutiquement acceptable. Ainsi, la co-administration de ces composés pourrait permettre d'améliorer les performances cognitives des patients par rapport à la simple administration d'un inhibiteur de l'acétylcholinestérase sans toutefois augmenter les effets délétères associés au traitement (en particulier, les troubles gastrointestinaux tels que la nausée ou la diarrhée, les céphalées ou la fatigue). Autrement dit, des traitements impliquant des doses thérapeutiques d'inhibiteur de l'acétylcholinestérase inférieures à celles classiquement utilisées en mono-administration deviennent donc envisageables, avec des performances cognitives équivalentes voire supérieures et des effets délétères moindres.

Cet effet, non prévisible, permet d'envisager l'utilisation des associations entre le 4-{3-[*cis*-hexahydrocyclopenta[*c*]pyrrol-2(1*H*)-yl]propoxy}benzamide, ou un de ses sels d'addition, et un inhibiteur de l'acétylcholinestérase dans le traitement des troubles cognitifs associés au vieillissement cérébral et aux maladies neurodégénératives. Les troubles cognitifs associés à la maladie d'Alzheimer et à la maladie de Parkinson sont particulièrement visés.

Préférentiellement, le 4-{3-[*cis*-hexahydrocyclopenta[*c*]pyrrol-2(1*H*)-yl]propoxy} benzamide est utilisé sous la forme d'un oxalate ou d'un chlorhydrate dans le cadre de l'invention.

Parmi les inhibiteurs de l'acétylcholinesterase selon l'invention, le donépézil, la rivastigmine et la galantamine sont particulièrement préférés. Le donépézil est préférentiellement utilisé sous la forme d'un chlorhydrate, la rivastigmine sous la forme d'un hydrogénotartrate, et la galantamine sous la forme d'un bromhydrate.

Plus particulièrement, l'association du 4-{3-[*cis*-hexahydrocyclopenta[*c*]pyrrol-2(1*H*)-yl]propoxy}benzamide et du donépézil est utilisée dans le traitement des troubles cognitifs associés à la maladie d'Alzheimer tandis que l'association du 4*-*{3-[*cis-*hexahydrocyclopenta[*c*]pyrrol-2(1*H*)-yl]propoxy}benzamide et de la rivastigmine est préférée dans le traitement des troubles cognitifs associés à la maladie de Parkinson. L'invention concerne donc l'utilisation de l'association entre le 4-{3-[*cis-*hexahydrocyclopenta[*c*]pyrrol-2(1*H*)-yl]propoxy}benzamide ou ses sels d'addition à un acide ou à une base pharmaceutiquement acceptable, et un inhibiteur de l'acétylcholinesterase, pour l'obtention de compositions pharmaceutiques destinées au traitement des troubles cognitifs associés au vieillissement cérébral et aux maladies neurodégénératives.

L'invention concerne également les compositions pharmaceutiques contenant l'association entre le 4-{3-[*cis*-hexahydrocyclopenta[*c*]pyrrol-2(1*H*)-yl]propoxy}benzamide ou ses sels d'addition à un acide ou à une base pharmaceutiquement acceptable, et un inhibiteur de l'acétylcholinesterase en combinaison avec un ou plusieurs excipients pharmaceutiquement acceptables.

Dans les compositions pharmaceutiques selon l'invention, la fraction massique en principes actifs (masse des principes actifs sur la masse totale de la composition) est préférentiellement comprise entre 5 et 50%.

Parmi les compositions pharmaceutiques selon l'invention, on retiendra plus particulièrement celles qui conviennent pour l'administration par voie orale, parentérale et notamment intraveineuse, per ou transcutanée, nasale, rectale, perlinguale, oculaire, respiratoire et plus spécifiquement les comprimés simples ou dragéifiés, les comprimés sublinguaux, les gélules, les glossettes, les capsules, les tablettes, les préparations injectables, les aérosols, les gouttes oculaires ou nasales, les suppositoires, les crèmes, pommades, gels dermiques, etc...

Outre le 4-{3-[*cis*-hexahydrocyclopenta[*c*]pyrrol-2(1*H*)-yl]propoxy}benzamide et le composé inhibiteur de l'acétylcholinesterase, les compositions pharmaceutiques selon l'invention contiennent un ou plusieurs excipients ou véhicules choisis parmi des diluants, des lubrifiants, des liants, des agents de désintégration, les stabilisants, les conservateurs, des absorbants, des colorants, des édulcorants, les aromatisants etc...

A titre d'exemple et de manière non limitative, on peut citer:
◆ *pour les diluants:* le lactose, le dextrose, le sucrose, le mannitol, le sorbitol, la cellulose, la glycérine,
◆ *pour les lubrifiants:* la silice, le talc, l'acide stéarique et ses sels de magnésium et de calcium, le polyéthylène glycol,
◆ *pour les liants:* le silicate d'aluminium et de magnésium, l'amidon, la gélatine, la tragacanthe, la méthylcellulose, la carboxyméthylcellulose de sodium et la polyvinylpyrrolidone,
◆ *pour les désintégrants:* l'agar, l'acide alginique et son sel de sodium, les mélanges effervescents.

Les composés de l'association peuvent être administrés de manière simultanée ou séquentielle. La voie d'administration préférée est la voie orale et les compositions pharmaceutiques correspondantes peuvent permettre la libération instantanée ou différée des principes actifs. Par ailleurs, les composés de l'association peuvent être administrés sous la forme de deux compositions pharmaceutiques distinctes, contenant chacune l'un des principes actifs, ou bien sous la forme d'une seule composition pharmaceutique, dans laquelle les principes actifs sont mélangés.

Les compositions pharmaceutiques préférées sont les comprimés.

La posologie utile varie selon le sexe, l'âge et le poids du patient, la voie d'administration, la nature de l'affection et des traitements éventuellement associés et s'échelonne entre 0,5 mg et 100 mg de 4-{3-[*cis*-hexahydrocyclopenta[*c*]pyrrol-2(1*H*)-yl]propoxy}benzamide par 24 heures, et plus préférentiellement 5 mg par jour. La dose de l'inhibiteur de l'acétylcholinesterase sera égale ou inférieure à celle utilisée lorsqu'il est administré seul. Dans le cas du donépézil, la posologie est comprise entre 0,5 mg et 30 mg par jour, les doses quotidiennes préférées étant de 5 et 10 mg pour le chlorhydrate de donépézil. Pour la rivastigmine, la posologie est comprise entre 1 mg et 20 mg par jour. Les doses quotidiennes préférées sont de 3 et 6 mg deux fois par jour lorsque la rivastigmine est administrée sous la forme d'un comprimé, tandis qu'elles sont de 4,6 mg et de 9,5 mg par jour lorsque le produit est présenté sous la forme d'un patch. Dans le cas de la galantamine, la posologie est comprise entre 1 et 30 mg par jour, les doses quotidiennes préférées étant de 16 et 24 mg.

Dans les modes de réalisation préférés de l'invention, l'association entre le 4-{3-[*cis-*hexahydrocyclopenta[*c*]pyrrol-2(1*H*)-yl]propoxy}benzamide (composé S) et le donépézil est administrée aux doses journalières suivantes :

| | composition 1 | composition 2 | composition 3 |
|---|---|---|---|
| Chlorhydrate du composé S (exprimé en équivalent base) | 2 mg | 5 mg | 20 mg |
| Chlorhydrate de donépézil | 10 mg | 10 mg | 10 mg |

### Composition pharmaceutique:

Formule de préparation pour 1000 comprimés dosés à 5 mg (en équivalent base) de chlorhydrate de 4-{3-[*cis*-hexahydrocyclopenta[*c*]pyrrol-2(1*H*)-yl]propoxy}benzamide et 10 mg de chlorhydrate de donépézil:

| | |
|---|---|
| Chlorhydrate de 4-{3-[*cis*-hexahydrocyclopenta[*c*]pyrrol-2(1*H*)-yl]propoxy}benzamide | |
| (exprimé en équivalent base) | 5 g |
| Chlorhydrate de donépézil | 10 g |
| Amidon de mais | 20 g |
| Maltodextrine | 7,5 g |
| Silice colloïdale | 0,2 g |
| Glycolate d'amidon de sodium | 3 g |
| Stéarate de magnésium | 1 g |
| Lactose | 55 g |

### EXEMPLE A:

### Expérience dans un modèle de mémoire épisodique, le test de discrimination contextuelle en série :

Les effets du 4-{3-[*cis*-hexahydrocyclopenta[*c*]pyrrol-2(1*H*)-yl]propoxy}benzamide et du donépézil (tous deux utilisés sous la forme d'un chlorhydrate), administrés seuls ou en combinaison, ont été étudiés en utilisant un test de discrimination contextuelle en série chez la souris C57B16 d'âge moyen 14-15 mois (n=12 par groupe) (Célérier et al, Learn Mem., 2004, 11(2), 196-204; Tronche et al., Behav. Brain Res., 2010, 215(2): 255-60).

Dans ce modèle, les souris d'âge moyen présentent par rapport aux souris jeunes un dysfonctionnement spécifique de la mémoire contextuelle, sans déficit de mémoire spatiale. Ce modèle est pertinent pour évaluer les effets des produits dans la maladie d'Alzheimer car les patients atteints de cette démence présentent également, et ce de manière très précoce, des troubles de mémoire épisodique contextuelle (Gold and Budson, Expert Rev Neurother., 2008, 8(12): 1879-1891).

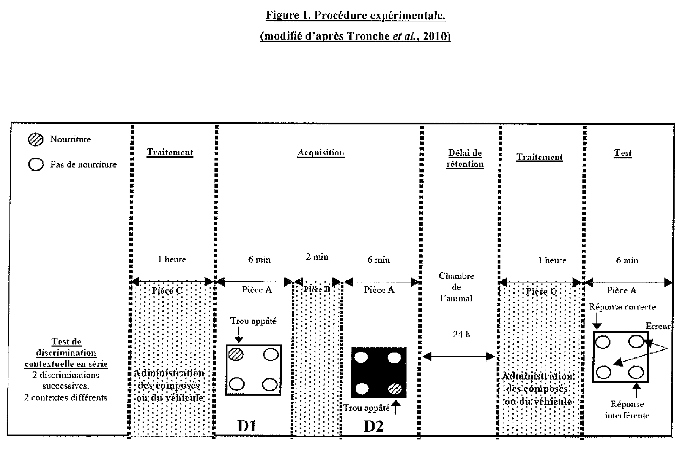

Les souris, placées dans une boîte à bords élevés, apprennent deux types de discriminations spatiales consécutives (D1: plancher blanc puis D2: plancher noir) sur un plancher à quatre trous, dans lequel seul un des trous est appâté, et ce de manière opposée entre D1 et D2 (voir Figure 1). Chaque discrimination est réalisée sur un plancher spécifique (blanc ou noir), qui constitue le contexte interne spécifique à chaque discrimination. 24h après l'étape d'apprentissage, les souris sont replacées sur le plancher contextuel blanc et sont mesurés :
- le pourcentage de réponses correctes (i.e. % d'inclinaisons de la tête dans le trou ayant été appâté pendant l'exercice d'acquisition sur le plancher blanc),
- le pourcentage de réponses interférentes (i.e. % d'inclinaisons de la tête dans le trou ayant été appâté pendant l'exercice d'acquisition sur le plancher noir, le dernier contexte présenté aux souris),
- et le pourcentage d'erreurs (i.e. % d'inclinaisons de la tête dans les deux trous n'ayant pas été appâtés pendant l'acquisition, ni sur le plancher blanc, ni sur le plancher noir) (voir Figure 1).

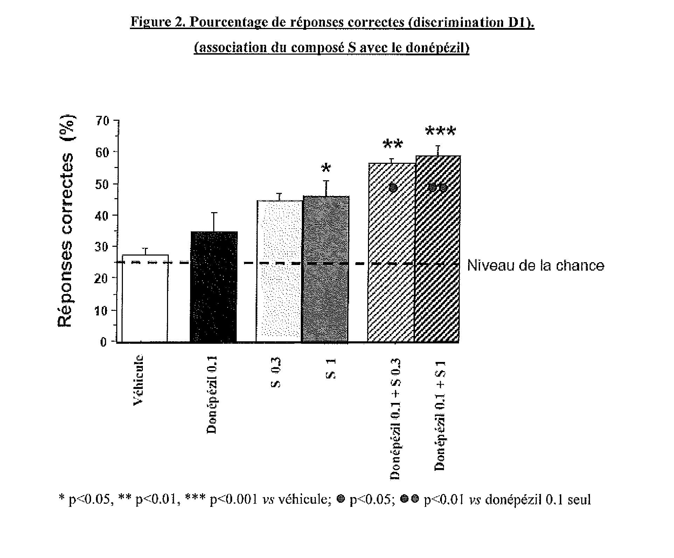

Les résultats montrent que ces souris d'âge moyen traitées avec le véhicule présentent un pourcentage de réponses correctes proches du niveau de la chance dans ce test sur plaques à 4 trous (≈ 25%). Après un traitement chronique de 9 jours de chlorhydrate de donépézil (0,1 mg/kg de base per os), aucune augmentation significative du pourcentage de réponses correctes n'est observée par rapport au véhicule (voir Figure 2). En revanche, le taux de réponses correctes augmente de plus de 60% par rapport au véhicule après un traitement chronique de 9 jours de chlorhydrate de 4-{3-[*cis*-hexahydrocyclopenta[*c*]pyrrol-2(1*H*)-yl]propoxylbenzamide aux doses de 0,3 et 1 mg/kg de base per os (composé appelé S sur la Figure 2). Enfin, l'administration de l'association du 4-{3-[*cis-*hexahydrocyclopenta[*c*]pyrrol-2(1*H*)-yl]propoxy}benzamide (0,3 et 1 mg/kg de base per os) avec le donépézil (0,1 mg/kg de base per os) conduit à une augmentation du taux de réponses correctes supérieure à 100% par rapport au véhicule seul. Ces résultats montrent une potentialisation nette des effets du donépézil en présence de 4-{3-[*cis-*hexahydrocyclopenta[*c*]pyrrol-2(1*H*)-yl]propoxy}benzamide.

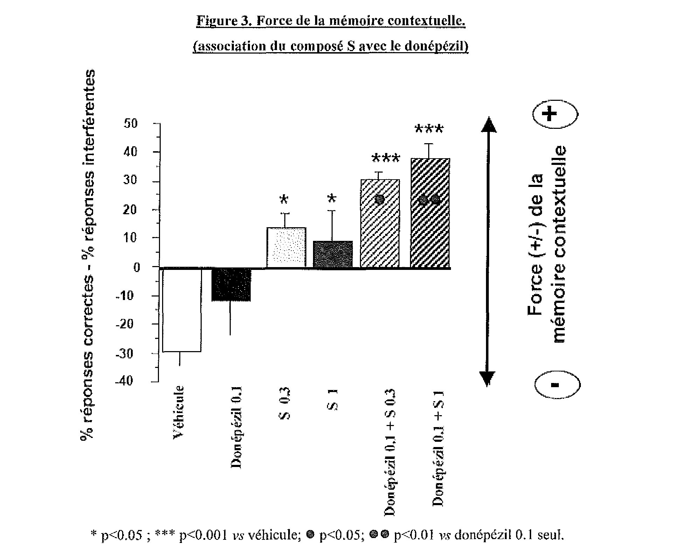

De plus, on a observé une très bonne corrélation entre l'augmentation du taux de réponses correctes et la diminution du taux de réponses interférentes, confirmant ainsi l'effet spécifique de chaque composé et de leur association sur la mémoire contextuelle. Ainsi, l'administration de l'association du 4-{3-[*cis*-hexahydrocyclopenta[*c*]pyrrol-2(1*H*)-yl]propoxy}benzamide (0,3 et 1 mg/kg de base per os) avec le donépézil (0,1 mg/kg de base per os) augmente significativement la force de la mémoire contextuelle (réponses correctes - réponses interférentes) par rapport à celle observée avec les composés seuls. Cette augmentation observée pour l'association ne peut s'expliquer par une simple additivité des effets des composés administrés seuls et montre une synergie d'activité des deux composés lorsqu'ils sont co-administrés (voir Figure 3).

Les résultats mettent clairement en évidence que l'administration de ces deux composés en association permet d'obtenir un effet synergique important tout à fait inattendu. Par ailleurs, les analyses pharmacocinétiques ont montré qu'il n'y avait aucune interaction de type pharmacocinétique entre les deux traitements qui pourrait justifier ou interférer avec l'effet synergique décrit ci-dessus.

### EXEMPLE B:

### Expérience avec la rivastigmine dans le même test de discrimination contextuelle en série :

Les effets du 4-{3-[*cis*-hexahydrocyclopenta[*c*]pyrrol-2(1*H*)-yl]propoxy}benzamide et de la rivastigmine (utilisée sous la forme d'un hydrogénotartrate), administrés seuls ou en combinaison, ont été étudiés en utilisant le test de discrimination contextuelle en série chez la souris C57B16 d'âge moyen décrit dans l'exemple précédent.

Dans ce modèle, il a été montré que les souris d'âge moyen présentent un déficit de mémoire contextuelle par rapport aux souris jeunes, dû au fait que le dernier contexte où elles ont appris la localisation du trou appâté (i.e. plancher noir) interfère de façon importante avec la mémoire du trou appâté dans le premier contexte présenté lors de l'acquisition (i.e. plancher blanc). De ce fait, les souris âgées ont des valeurs de force de la mémoire contextuelle (réponses correctes - réponses interférentes) négatives puisque le pourcentage de réponses interférentes est supérieur au pourcentage de réponses correctes. A l'inverse, les souris jeunes présentent une force de la mémoire contextuelle positive (Tronche et al., Behav. Brain Res., 2010, 215(2): 255-60).

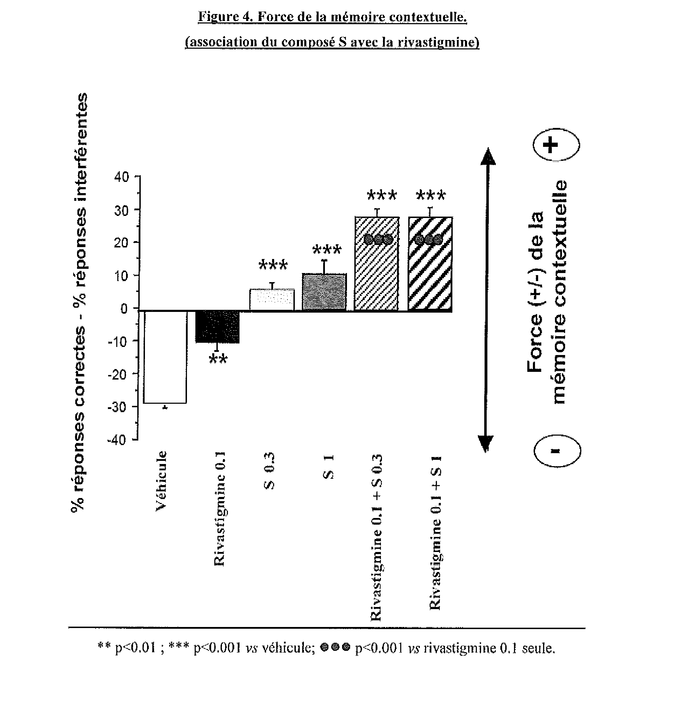

Les résultats de cette étude confirment, comme dans l'exemple précédent, le déficit de mémoire contextuelle des souris d'âge moyen: les souris traitées par le véhicule montrent en effet une force de la mémoire contextuelle négative de -28% (voir Figure 4). Après un traitement chronique de 9 jours avec la rivastigmine à la dose de 0,1 mg/kg de base per os, une légère augmentation de la force de la mémoire contextuelle est observée par rapport au véhicule (-9% versus -28%), mais celle-ci reste négative, le pourcentage de réponses interférentes étant toujours supérieur au pourcentage de réponses correctes, faisant donc considérer la dose de 0,1 mg/kg de rivastigmine comme une dose subactive.

De même, la force de la mémoire contextuelle n'augmente que légèrement par rapport au véhicule après un traitement chronique de 9 jours de chlorhydrate de 4-{3-[*cis-*hexahydrocyclopenta[*c*]pyrrol-2(1*H*)-yl]propoxy}benzamide aux doses de 0,3 et de 1 mg/kg de base per os (+6% et +10% versus -28%, respectivement). En revanche, l'administration de l'association de la rivastigmine (dose subactive de 0,1 mg/kg de base per os) avec le 4-{3-[*cis*-hexahydrocyclopenta[*c*]pyrrol-2(1*H*)-yl]propoxy}benzamide (0,3 ou 1 mg/kg de base per os) conduit à une augmentation importante de la force de la mémoire contextuelle, qui devient alors significativement supérieure (% réponses correctes > % réponses interférentes), d'une part vis-à-vis de la valeur obtenue avec le véhicule, et d'autre part, vis-à-vis de celle obtenue pour la rivastigmine seule (+28% et +28% versus - 9%, respectivement). Ces résultats montrent une potentialisation nette des effets de la rivastigmine à dose subactive en présence de doses actives de 4-{3-[*cis-*hexahydrocyclopenta[*c*]pyrrol-2(1*H*)-yl]propoxy}benzamide, qui se traduit par un accroissement de la performance mnésique des souris traitées par l'association.

Dans ce deuxième exemple également, l'augmentation de la force de la mémoire contextuelle observée pour les deux associations ne peut pas s'expliquer par une simple additivité des effets des composés administrés seuls, et montre une synergie d'activité tout a fait inattendue des deux composés lorsqu'ils sont co-administrés.

Par ailleurs, les analyses pharmacocinétiques ont mis en évidence qu'il n'y avait aucune interaction de type pharmacocinétique entre les deux traitements qui pourrait justifier ou interférer avec l'effet synergique décrit ci-dessus.

En conclusion, les résultats présentés ci-dessus mettent en évidence une synergie d'activité entre le 4-{3-[*cis*-hexahydrocyclopenta[*c*]pyrrol-2(1*H*)-yl]propoxy}benzamide et la rivastigmine en terme de performances cognitives, et ce sans interaction pharmacocinétique.

### EXEMPLE C:

### Expérience avec la galantamine dans le même test de discrimination contextuelle en série:

Les effets du 4-{3-[*cis*-hexahydrocyclopenta[*c*]pyrrol-2(1*H*)-yl]propoxy}benzamide et de la galantamine (utilisée sous la forme d'un bromhydrate), administrés seuls ou en combinaison, ont également été étudiés en utilisant le test de discrimination contextuelle en série chez la souris C57B16 d'âge moyen.

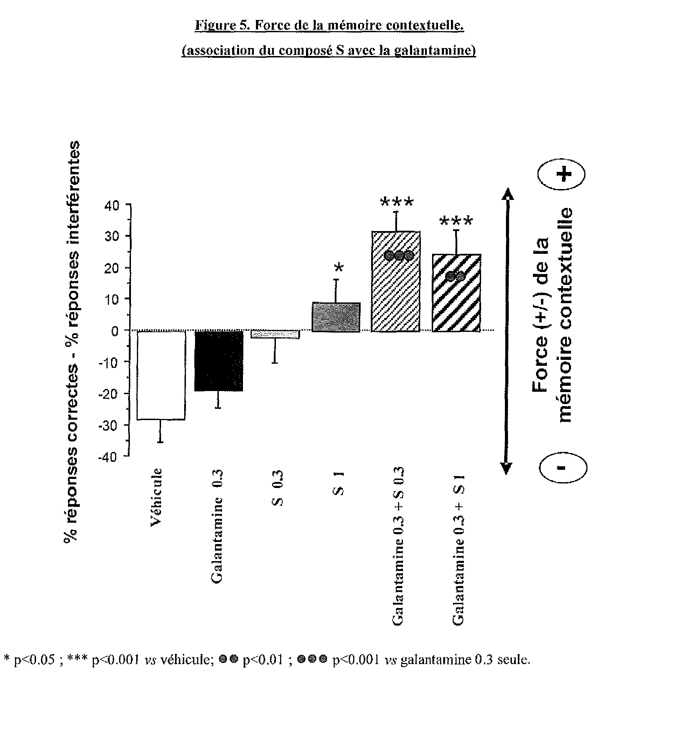

Les résultats de cette étude confirment, comme dans les exemples précédents, le déficit de mémoire contextuelle des souris d'âge moyen: les souris traitées par le véhicule montrent en effet une force de la mémoire contextuelle négative de -28% (voir Figure 5).

Après un traitement chronique de 9 jours avec la galantamine à la dose de 0,3 mg/kg de base per os, on n'observe aucune augmentation significative de la force de la mémoire contextuelle par rapport au véhicule (-18% versus -28%), qui reste fortement négative (% réponses interférentes > % réponses correctes) ; la dose de 0,3 mg/kg de galantamine est donc subactive. La force de la mémoire contextuelle n'augmente pas non plus de manière significative par rapport au véhicule après un traitement chronique de 9 jours de chlorhydrate de 4-{3-[*cis*-hexahydrocyclopenta[*c*]pyrrol-2(1*H*)-yl]propoxy}benzamide à la dose de 0.3 mg/kg de base per os (-3% versus -28%, respectivement). Par ailleurs, elle n'augmente que légèrement par rapport au véhicule (+9% versus -28%) après un traitement chronique de 9 jours de chlorhydrate de 4-{3-[*cis*-hexahydrocyclopenta[*c*]pyrrol-2(1*H*)-yl]propoxy}benzamide à la dose de 1 mg/kg de base per os. En revanche, l'administration de l'association du 4-{3-[*cis*-hexahydrocyclopenta[*c*]pyrrol-2(1*H*)-yl]propoxy}benzamide (0,3 et 1 mg/kg de base per os) avec la galantamine (dose subactive de 0,3 mg/kg de base per os) conduit à une augmentation importante de la force de la mémoire contextuelle, qui devient significativement supérieure par rapport à la valeur obtenue avec le véhicule seul (+31% et +24% versus -28%, respectivement). Ces résultats montrent une potentialisation nette des effets de la galantamine à dose subactive en présence d'une dose subactive ou légèrement active de 4-{3-[*cis*-hexahydrocyclopenta[*c*]pyrrol-2(1*H*)-yl]propoxy}benzamide, qui se traduit par un accroissement de la performance mnésique des souris traitées par l'association.

Là encore, dans ce troisième exemple d'association avec un inhibiteur de l'acétylcholinestérase, l'augmentation de la force de la mémoire contextuelle observée ne peut pas s'expliquer par une simple additivité des effets des composés administrés seuls. En conclusion, les résultats présentés ci-dessus mettent en évidence une synergie d'activité entre le 4-{3-[*cis*-hexahydrocyclopenta[*c*]pyrrol-2(1*H*)-yl]propoxy}benzamide et la galantamine en terme de performances cognitives.

### EXEMPLE D:

### Test d'observation primaire d'Irwin:

Les effets en terme de sécurité du 4-{3-[*cis*-hexahydrocyclopenta[*c*]pyrrol-2(1*H*)-yl]propoxy}benzamide et du donépézil (tous deux sous la forme d'un chlorhydrate), administrés seuls ou en association, ont été étudiés en utilisant le test d'observation primaire d'Irwin chez la souris C57B16 (n=4 individus par groupe).

Les changements comportementaux, les symptômes physiologiques et neurotoxiques, la température rectale, ainsi que le diamètre de la pupille ont été enregistrés suivant une grille d'observation standardisée, dérivée de celle d'Irwin.

Il a été observé que le 4-{3-[*cis*-hexahydrocyclopenta[*c*]pyrrol-2(1*H*)-yl]propoxy} benzamide (0,3 et 1 mg/kg de base per os) et le donépézil (0,1 et 0,3 mg/kg de base per os) donnés seuls ou en co-administration n'induisaient aucun changement observable dans le test d'Irwin chez la souris. A plus forte dose (1 mg/kg de base per os), le donépézil administré seul diminue la réactivité au toucher et induit une légère sédation. Aucune potentialisation des effets délétères du donépézil à 1 mg/kg n'est observée lorsqu'il est co-administré avec le 4-{3-[*cis*-hexahydrocyclopenta[*c*]pyrrol-2(1*H*)-yl]propoxy}benzamide (0,3 et 1 mg/kg de base per os). Au contraire, quand le 4-{3-[*cis-*hexahydrocyclopenta[*c*]pyrrol-2(1*H*)-yl]propoxy}benzamide est co-administré à la plus forte dose (1 mg/kg de base per os) avec le donépézil à 1 mg/kg p.o., aucune sédation n'est observée suggérant que le 4-{3-[*cis*-hexahydrocyclopenta[*c*]pyrrol-2(1*H*)-yl]propoxy}benzamide antagonise la sédation induite par le donépézil à 1 mg/kg p.o.

En conclusion, les résultats présentés ci-dessus mettent en évidence une synergie d'activité entre le 4-{3-[*cis*-hexahydrocyclopenta[*c*]pyrrol-2(1*H*)-yl]propoxy}benzamide et le donépézil en terme de performances cognitives, et ce avec un bon profil de sécurité et sans interaction pharmacocinétique.

## Revendications

1. Association entre le 4-{3-[*cis*-hexahydrocyclopenta[*c*]pyrrol-2(1*H*)-yl]propoxy}benzamide de formule (I): 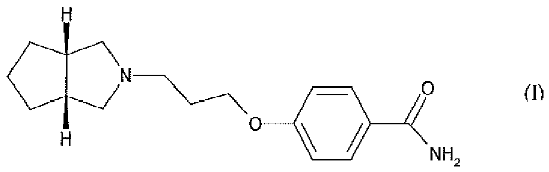 ou un de ses sels d'addition à un acide ou à une base pharmaceutiquement acceptable, et un inhibiteur de l'acétylcholinesterase.

2. Association selon la revendication 1 **caractérisée en ce que** le 4-{3-[*cis-*hexahydrocyclopenta[*c*]pyrrol-2(1*H*)-yl]propoxy}benzamide est utilisé sous la forme d'un oxalate ou d'un chlorhydrate.

3. Association selon la revendication 1 ou 2 dans laquelle l'inhibiteur de l'acétylcholinesterase est le donépézil, la rivastigmine ou la galantamine.

4. Association selon l'une des revendications 1 à 3 dans laquelle le donépézil est utilisé sous la forme d'un chlorhydrate, la rivastigmine sous la forme d'un hydrogénotartrate, et la galantamine sous la forme d'un bromhydrate.

5. Association selon l'une des revendications 1 à 4 **caractérisée en ce que** le 4-{3-[*cis-*hexahydrocyclopenta[*c*]pyrrol-2(1*H*)-yl]propoxy}benzamide est administré sous la forme d'un chlorhydrate à la dose journalière de 2 mg, 5 mg ou 20 mg (exprimée en
équivalent base), et **en ce que** l'inhibiteur de l'acétylcholinesterase est le chlorhydrate de donépézil administré à la dose journalière de 10 mg.

6. Composition pharmaceutique contenant comme principe actif le 4-{3-[*cis-*hexahydrocyclopenta[*c*]pyrrol-2(1*H*)-yl]propoxy}benzamide, ou un de ses sels d'addition à un acide ou à une base pharmaceutiquement acceptable, en association avec un inhibiteur de l'acétylcholinesterase selon l'une des revendications 1 à 4 en combinaison avec un ou plusieurs excipients pharmaceutiquement acceptables.

7. Composition pharmaceutique selon la revendication 6 pour son utilisation dans le traitement des troubles cognitifs associés au vieillissement cérébral et aux maladies neurodégénératives.

8. Composition pharmaceutique selon la revendication 7 pour son utilisation dans le traitement des troubles cognitifs associés à la maladie d'Alzheimer et à la maladie de Parkinson.

9. Composition pharmaceutique selon la revendication 6 **caractérisée en ce que** le 4-{3-[*cis*-hexahydrocyclopenta[*c*]pyrrol-2(1*H*)-yl]propoxy}benzamide est administré sous la forme d'un chlorhydrate à la dose journalière de 2 mg, 5 mg ou 20 mg (exprimée en équivalent base), et **en ce que** l'inhibiteur de l'acétylcholinesterase est le chlorhydrate de donépézil administré à la dose journalière de 10 mg.

10. Utilisation d'une association selon l'une des revendications 1 à 5 pour la fabrication d'un médicament destiné au traitement des troubles cognitifs associés au vieillissement cérébral et aux maladies neurodégénératives.

11. Utilisation d'une association selon l'une des revendications 1 à 5 pour la fabrication d'un médicament destiné au traitement des troubles cognitifs associés à la maladie d'Alzheimer et à la maladie de Parkinson.

12. Association selon l'une des revendications 1 à 5 entre le 4-{3-[*cis-*hexahydrocyclopenta[*c*]pyrrol-2(1*H*)-yl]propoxy}benzamide ou un de ses sels d'addition à un acide ou à une base pharmaceutiquement acceptable, et le donépézil pour son utilisation dans le traitement des troubles cognitifs associés à la maladie d'Alzheimer.

13. Association selon l'une des revendications 1 à 5 entre le 4-{3-[*cis-*hexahydrocyclopenta[*c*]pyrrol-2(1*H*)-yl]propoxy}benzamide ou un de ses sels d'addition à un acide ou à une base pharmaceutiquement acceptable, et la rivastigmine pour son utilisation dans le traitement des troubles cognitifs associés à la maladie de Parkinson.

14. Utilisation d'une association selon l'une des revendications 1 à 5 entre le 4-{3-[*cis-*hexahydrocyclopenta[*c*]pyrrol-2(1*H*)-yl]propoxy}benzamide ou un de ses sels d'addition à un acide ou à une base pharmaceutiquement acceptable, et le donépézil pour la fabrication d'un médicament destiné au traitement des troubles cognitifs associés à la maladie d'Alzheimer.

15. Utilisation d'une association selon l'une des revendications 1 à 5 entre le 4-{3-[*cis-*hexahydrocyclopenta[*c*]pyrrol-2(1*H*)-yl]propoxy}benzamide ou un de ses sels d'addition à un acide ou à une base pharmaceutiquement acceptable, et la rivastigmine pour la fabrication d'un médicament destiné au traitement des troubles cognitifs associés à la maladie de Parkinson.
